(19) Europäisches Patentamt
European Patent Office
Office européen des brevets

(11) Publication number: **0 051 326**
**B1**

(12) **EUROPEAN PATENT SPECIFICATION**

(45) Date of publication of patent specification: **04.07.84**

(51) Int. Cl.³: **C 07 C 15/00,** C 07 C 1/04, B 01 J 29/28

(21) Application number: **81201126.0**

(22) Date of filing: **09.10.81**

(54) **Process for the preparation of hydrocarbons.**

(30) Priority: **30.10.80 NL 8005952**

(43) Date of publication of application:
**12.05.82 Bulletin 82/19**

(45) Publication of the grant of the patent:
**04.07.84 Bulletin 84/27**

(84) Designated Contracting States:
**BE DE FR GB IT NL**

(56) References cited:
**GB - A - 1 555 928**
**GB - A - 2 037 315**
**GB - A - 2 037 316**
**US - A - 4 086 262**
**US - A - 4 180 516**
**US - A - 4 188 336**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Boersma, Michael Adriaan Maria**
**Badhuisweg 3**
**NL-1031 CM Amsterdam (NL)**
Inventor: **Post, Martin Franciscus Maria**
**Badhuisweg 3**
**NL-1031 CM Amsterdam (NL)**
Inventor: **Schaper, Lambert**
**Badhuisweg 3**
**NL-1031 CM Amsterdam (NL)**

(74) Representative: **Puister, Antonius Tonnis, Mr. et al,**
**P.O. Box 302**
**NL-2501 CH The Hague (NL)**

**EP 0 051 326 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The invention relates to a process for the preparation of an aromatic hydrocarbon mixture from a mixture of carbon monoxide and hydrogen.

From GB—A—2,037,315 it is known, that mixtures of carbon monoxide and hydrogen can be converted into aromatic hydrocarbon mixtures using a mixture of two catalysts, of which one has the capability of catalysing the conversion of an $H_2/CO$ mixture into acyclic oxygen-containing hydrocarbons, and the other is a crystalline aluminium silicate having the capability of catalysing the conversion of acyclic oxygen containing hydrocarbons into aromatic hydrocarbons. The said crystalline aluminium silicates are characterized in that after one hour's calcining in air at 500°C they have the following properties:

(a) thermally stable up to a temperature of at least 600°C,
(b) an X-ray powder diffraction pattern showing the reflections given in Table A, and
(c) in the formula which gives the composition of the silicate, expressed in moles of the oxides, the $SiO_2/Al_2O_3$ molar ratio (for sake of brevity further designated m in this patent application) is higher than 200.

TABLE A

| d(Å) | relative intensity | d(Å) | relative intensity |
|---|---|---|---|
| 11.1 | 100 | 3.84 (D) | 57 |
| 10.0 (D) | 70 | 3.70 (D) | 31 |
| 8.93 | 1 | 3.63 | 16 |
| 7.99 | 1 | 3.47 | 1 |
| 7.42 | 2 | 3.43 | 5 |
| 6.68 | 7 | 3.34 | 2 |
| 6.35 | 11 | 3.30 | 5 |
| 5.97 | 17 | 3.25 | 1 |
| 5.70 | 7 | 3.05 | 8 |
| 5.56 | 10 | 2.98 | 11 |
| 5.35 | 2 | 2.96 | 3 |
| 4.98 (D) | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |
| 4.00 | 4 | | |

(D)=doublet

The expression "thermally stable" up to a temperature of at least t°C, used in this patent application in connection with crystalline silicates, means that if the silicate is heated up to a temperature of t°C the X-ray powder diffraction pattern remains substantially unchanged.

The crystalline silicates used in the catalyst mixtures are prepared from an aqueous starting mixture containing the following compounds: one or more compounds of an alkali metal (M), a tetrapropylammonium compound, one or more silicon compounds and one or more aluminium compounds.

The preparation of the crystalline silicates takes place by maintaining the mixture at elevated temperature until the silicate has formed, separating it from the mother liquor and calcining it.

The average crystallite size of the silicate is preferably below 3000 nm.

The Applicant has carried out an investigation concerning the use of the crystalline aluminium silicates prepared as described above as the catalyst component in the above-mentioned catalyst mixtures to be employed in the preparation of an aromatic hydrocarbon mixture from an $H_2/CO$ mixture. It was found in this investigation that the behaviour of these catalyst mixtures as regards their activity, stability, $C_5^+$ selectivity and durene production is determined to a high degree by the $SiO_2/Al_2O_3$ molar ratio (m) and the average crystallite size (d) of the crystalline aluminium silicate present in the catalyst mixture. The said durene production is especially important if the aim is to use the aromatic hydrocarbon mixtures prepared as gasoline. In view of the high melting point of durene it is desirable that its concentration in the aromatic hydrocarbon mixture be as low as possible.

The investigation has shown these catalyst mixtures to have a high activity, stability and $C_5^+$ selectivity and a low durene production, if the crystalline aluminium silicate present therein has an m between 200 and 750 and a d between 400 and 1500 nm. In view of the desired m-value, the silicon and aluminium compounds should be present in the aqueous mixture from which the silicates are

prepared in such a ratio, expressed in moles of the oxides, that the requirement $SiO_2:Al_2O_3$=150—1500 is satisfied.

The present patent application therefore relates to a process for the preparation of an aromatic hydrocarbon mixture in which an $H_2$/CO mixture is contacted with a mixture of two catalysts of which one (catalyst X) has the capability of catalysing the conversion of an $H_2$/CO mixture into acyclic oxygen-containing hydrocarbons and the other (catalyst Y) is a crystalline aluminium silicate which, after one hour's calcining in air at 500°C, has the following properties:

(a) thermally stable up to a temperature of at least 600°C,
(b) an X-ray powder diffraction pattern showing the reflections given in Table A,
(c) an m-value above 200, and
(d) a d-value below 3000 nm, which crystalline aluminium silicate has been prepared by maintaining an aqueous mixture containing the following compounds: one or more compounds of an alkali metal (M), a tetrapropylammonium compound, one or more silicon compounds and one or more aluminium compounds, at elevated temperature until the crystalline silicate has been formed and subsequently separating this from the mother liquor and calcining it, characterized in that the crystalline aluminium silicate has been prepared from an aqueous mixture in which mixture the compounds are present in the following ratios, expressed in moles of the oxides:

$M_2O:SiO_2$=0.01—0.35,
$[(C_3H_7)_4N]_2O:SiO_2$=0.01—0.4,
$SiO_2:Al_2O_3$=150—1500, and
$H_2O:SiO_2$=5—65,

in that in the formula representing the composition of the silicate, the $SiO_2/Al_2O_3$ molar ratio is 200—750, and in that the silicate has an average crystallite size of 400—1500 nm.

In the process according to the invention the starting material is an $H_2$/CO mixture. Such a mixture can very suitably be prepared by steam gasification of a carbon-containing material. Examples of such materials are brown coal, anthracite, coke, crude mineral oil and fractions therefrom, and oils produced from tar sand and bituminous shale. The steam gasification is preferably carried out at a temperature between 900 and 1500°C and a pressure between 10 and 50 bar. The preferred starting material in the process according to the invention is an $H_2$/CO mixture whose molar ratio lies between 0.25 and 1.0.

The process according to the invention is preferably carried out at a temperature of 200—500°C and in particular of 300—450°C, a pressure of 1—150 bar and in particular of 5—100 bar and a space velocity of 50—5000 and in particular of 300—3000 Nl gas/l catalyst/h.

In the process according to the invention as well as in the known process a mixture of two catalysts is used, which for convenience are designated catalyst X and catalyst Y. Catalyst X is the one which is capable of catalysing the conversion of an $H_2$/CO mixture into acyclic oxygen-containing hydrocarbons and catalyst Y is the crystalline aluminium silicate. Catalysts that are preferably used as X-catalysts are those which are capable of converting an $H_2$/CO mixture into substantially methanol and/or dimethylether. Very suitable for the present purpose are catalysts containing zinc together with chromium. When using such a catalyst, it is preferred to choose one in which the atomic percentage of zinc, based on the sum of zinc and chromium, is at least 60% and in particular 60—80%. According to the invention it is preferred to use catalyst mixtures containing per part by weight of catalyst Y, 2.5—12.5 parts by weights of catalyst X. Catalyst mixtures in which a catalyst X is present which contains zinc together with chromium and in which the atomic percentage of zinc, based on the sum of zinc and chromium, is between 60 and 80%, show a very high activity, when they contain per part by weight of catalyst Y, 4—8 parts by weight of such a catalyst X.

In the preparation of the aluminium silicates that are used in the process according to the invention the silicon compound is preferably amorphous silica and the aluminium compound is preferably $NaAlO_2$ or amorphous alumina.

The m-value can be controlled by means of the molar ratio of $SiO_2$ and $Al_2O_3$ in the starting mixture, in the sense that silicates with a higher m-value are obtained according as the molar ratio of $SiO_2$ to $Al_2O_3$ in the starting mixture is chosen higher. The d-value can be controlled by means of the molar ratio of $[(C_3H_7)_4N]_2O$ to $SiO_2$ in the starting mixture, in the sense that silicates with a lower d-value are obtained according as the molar ratio of $[(C_3H_7)_4N]_2O$ to $SiO_2$ in the starting mixture is chosen higher.

The aluminium silicates that are used in the process according to the invention are prepared from an aqueous mixture containing one or more alkali metal compounds. The preferred alkali metal compound in the preparation of the aluminium silicates is a sodium compound. The alkali metal ions present in the aqueous mixture from which the aluminium silicates are prepared find their way at least partly in the silicates prepared. With the aid of suitable exchange methods they can be replaced by other cations such as hydrogen ions or ammonium ions. The aluminium silicates used in the catalyst mixtures

preferably have an alkali metal content of less than 0.1% w and in particular less than 0.05% w. If desired, a binder material such as bentonite or kaolin may be incorporated into the catalyst mixtures.

The process according to the invention can very suitably be carried out by conducting the feed in upward or downward direction through a vertically mounted reactor in which a fixed or a moving bed of the catalyst mixture concerned is present.

The process according to the invention can also very suitably be used as the first step of a two-step process for the conversion of $H_2/CO$ mixtures into hydrocarbon mixtures. In this case carbon monoxide and hydrogen present in the reaction product of the first step are, if desired together with other components of this reaction product, contacted in a second step with a catalyst containing one or more metal components with catalytic activity for the conversion of an $H_2/CO$ mixture into acyclic hydrocarbons, which metal components have been chosen from the group formed by cobalt, nickel and ruthenium, on the understanding that if the feed for the second step has an $H_2/CO$ molar ratio of less than 1.5, water is added to this feed and that in the second step a bifunctional catalyst or catalyst combination is used which, in addition to the metal components with catalytic activity for the conversion of an $H_2/CO$ mixture into acyclic hydrocarbons, also contains one or more metal components with catalytic activity for the conversion of an $H_2O/CO$ mixture into an $H_2/CO_2$ mixture.

Further, the process according to the invention can very suitably be used as the first step of a three-step process for the preparation of, inter alia, middle distillates from an $H_2/CO$ mixture. In this case carbon monoxide and hydrogen present in the reaction product of the first step, if desired together with other components of this reaction product, are contacted in a second step with a catalyst that contains 10—40 pbw cobalt and 0.25—5 pbw zirconium, titanium or chromium per 100 pbw silica and which has been prepared by impregnating a silica carrier with one or more aqueous solutions of salts of cobalt and zirconium, titanium or chromium, followed by drying the composition, calcining at 350—700°C and reducing at 200—350°C, on the understanding that if the feed for the second step has an $H_2/CO$ molar ratio of less than 1.5, water is added to the feed and that the cobalt-impregnation catalyst is used in combination with a CO shift catalyst. Of the reaction product of the second step at least the part whose initial boiling point lies above the final boiling point of the heaviest middle distillate desired as the end product, is subjected in a third step to a catalytic hydrotreatment.

The invention will now be explained with reference to the following example.

Example

Thirteen crystalline aluminium silicates (silicates 1—13) were prepared by heating mixtures of NaOH, $(C_3H_7)_4NOH$, amorphous silica (containing 300 ppmw Al) and $NaAlO_2$ for 24 hours at 150°C in water in an autoclave under autogeneous pressure, either with stirring or not. Further, two crystalline aluminium silicates (silicates 14 and 15) were prepared, substantially in the same way as the silicates 1—13, the differences being that in the preparation of silicate 14 use was made of amorphous alumina instead of $NaAlO_2$ and that in the preparation of silicate 15 use was made of $C_4H_9NH_2$ instead of $(C_3H_7)_4NOH$. Finally, a crystalline silicate (silicate 16) was prepared, substantially in the same way as the silicates 1—13, the difference being that in the preparation of silicate 16 no use was made of $NaAlO_2$. After the reaction mixtures had cooled down, the silicates formed were filtered off, washed with water until the pH of the wash water was about 8, dried at 120°C and calcined at 500°C. The silicates 1—16 had the following properties:

(a) thermally stable up to a temperature of at least 800°C,
(b) an X-ray powder diffraction pattern as given in Table A, and
(c) values for m and d as given in Table B.

TABLE B

| silicate No. | m | d, nm |
|---|---|---|
| 1 | 323 | 1000 |
| 2 | 343 | 1000 |
| 3 | 343 | 1200 |
| 4 | 357 | 450 |
| 5 | 310 | 100 |
| 6 | 280 | 200 |
| 7 | 330 | 2000 |
| 8 | 700 | 150 |
| 9 | 110 | 1000 |
| 10 | 90 | 350 |
| 11 | 600 | 500 |
| 12 | 205 | 700 |
| 13 | 225 | 1250 |
| 14 | 255 | 300 |
| 15 | 225 | 1400 |
| 16 | 2200 | 150 |

The molar composition of the aqueous mixtures from which the silicates 1—16 were prepared can be represented as follows:

$$x\ Na_2O.\ y\ [(C_3H_7)_4N]_2O.\ z\ C_4H_9NH_2.\ p\ Al_2O_3.\ 25\ SiO_2.\ 450\ H_2O,$$

wherein x, y, z and p have the values given in Table C. Table C also gives for each silicate whether in the preparation stirring took place or not.

TABLE C

| Silicate No. | Prepared from silicate No. | x | y | z | p | stirred |
|---|---|---|---|---|---|---|
| 17 | 1 | 1 | 0.5 | — | 0.091 | yes |
| 18 | 2 | 1 | 1.5 | — | 0.079 | yes |
| 19 | 3 | 1 | 2.0 | — | 0.079 | no |
| 20 | 4 | 1 | 3.25 | — | 0.058 | yes |
| 21 | 5 | 3 | 4.5 | — | 0.039 | no |
| 22 | 6 | 1 | 4.5 | — | 0.071 | yes |
| 23 | 7 | 1 | 0.375 | — | 0.091 | yes |
| 24 | 8 | 3 | 4.5 | — | 0.020 | no |
| 25 | 9 | 1 | 1.5 | — | 0.21 | yes |
| 26 | 10 | 1 | 4.5 | — | 0.18 | yes |
| 27 | 11 | 1 | 3.0 | — | 0.033 | no |
| 28 | 12 | 3 | 4.5 | — | 0.050 | yes |
| 29 | 13 | 3 | 4.5 | — | 0.042 | yes |
| 30 | 14 | 1 | 4.5 | — | 0.071 | yes |
| 31 | 15 | 2 | — | 10 | 0.11 | no |
| 32 | 16 | 3 | 4.5 | — | 0.008 | no |

From the silicates 1—16 the silicates 17—32 were prepared, respectively, by boiling the silicates 1—16 with 1.0 molar $NH_4NO_3$ solution, washing with water, boiling again with 1.0 molar $NH_4NO_3$ solution, drying at 120°C and calcining at 500°C. Subsequently, 18 catalyst mixtures (catalyst mixtures A—R) were prepared by mixing a $ZnO—Cr_2O_3$ composition with each of the silicates 17—32. The atomic Zn percentage of the $ZnO—Cr_2O_3$ composition, based on the sum of Zn and Cr, was 70%. The catalyst mixtures A—M contained per pbw silicate, 10 pbw of the $ZnO—Cr_2O_3$ composition. The catalyst mixtures N and O contained per pbw silicate, 5 and 3.5 pbw, respectively of the $ZnO—Cr_2O_3$ composition.

The catalyst mixtures A—R were tested for the preparation of an aromatic hydrocarbon mixture from an $H_2/CO$ mixture. The test was carried out in a 50-ml reactor in which a fixed catalyst bed with a volume of 7.5 ml was present. In eighteen experiments in $H_2/CO$ mixture with an $H_2/CO$ molar ratio of 0.5 was conducted over each of the catalyst mixtures A—R at a temperature of 375°C, a pressure of 60 bar and a space velocity of 1000 $Nl.l^{-1}.h^{-1}$. In all the experiments the aromatics content of the $C_5^+$ fraction, averaged over 100 hours, was higher than 40% w. The other results of the experiments have been listed in Table D.

TABLE D

| Experiment No. | Cat. mixture No. | Silicate No. | Conversion of the synthesis gas, %v after 10 h. (C 10) | after 100 h. (C 100) | Stability expressed as difference between C 10 and C 100 | $C_5^+$ selectivity averaged over 100 h, %w | Durene content in $C_5^+$ product averaged over 100 h, %w |
|---|---|---|---|---|---|---|---|
| 1 | A | 17 | 62 | 53 | 9 | 75 | 8.9 |
| 2 | B | 18 | 58 | 52 | 6 | 75 | 8.4 |
| 3 | C | 19 | 56 | 50 | 6 | 77 | 9.1 |
| 4 | D | 20 | 60 | 56 | 4 | 75 | 11.6 |
| 5 | E | 21 | 58 | 58 | 0 | 75 | 25.6 |
| 6 | F | 22 | 66 | 63 | 3 | 70 | 20.2 |
| 7 | G | 23 | 50 | 34 | 16 | 80 | 5.6 |
| 8 | H | 24 | 56 | 55 | 1 | 79 | 21.9 |
| 9 | I | 25 | 68 | 56 | 12 | 65 | 10.2 |
| 10 | J | 26 | 68 | 64 | 4 | 62 | 14.6 |
| 11 | K | 27 | 52 | 48 | 4 | 78 | 12.0 |
| 12 | L | 28 | 63 | 62 | 1 | 74 | 13.0 |
| 13 | M | 29 | 64 | 57 | 7 | 72 | 9.5 |
| 14 | N | 29 | 71 | 68 | 3 | 70 | 10.0 |
| 15 | O | 29 | 65 | 63 | 2 | 67 | 10.0 |
| 16 | P | 30 | 64 | 61 | 3 | 70 | 21.7 |
| 17 | Q | 31 | 55 | 15 | 40 | 75 | 7.0 |
| 18 | R | 32 | 30 | 28⊕) | 2⊕) | 78⊕) | 11.9⊕) |

⊕) Experiment finished after 30 h.

Of the experiments listed in Table D only the experiments 1—4 and 11—15 are experiments according to the invention. In these experiments use was made of catalyst mixtures containing silicates with an m between 200 and 750 and a d between 400 and 1500 nm, which silicates had been prepared using a tetrapropylammonium compound. These catalyst mixtures all showed a high activity, stability and $C_5^+$ selectivity and a low durene production. The experiments 5—10 and 16—18 are outside the scope of the invention. They have been included in this patent application for comparison.

In the experiments 5, 6, 8, and 16 use was made of catalyst mixtures containing silicates with a d<400 nm. These catalyst mixtures showed too high a durene production.

In experiment 7 use was made of a catalyst mixture containing a silicate with a d 1500 nm. The stability of this catalyst mixture was too low.

In experiment 9 use was made of a catalyst mixture containing a silicate with an m 200. This catalyst mixture showed too low a stability and too low a $C_5^+$ selectivity.

In experiment 10 use was made of a catalyst mixture containing a silicate with an m 200 and a d 400 nm. The $C_5^+$ selectivity of this catalyst mixture was too low.

In experiment 17 use was made of a catalyst mixture whose silicate component had been prepared using an amine instead of a tetrapropylammonium compound. This catalyst mixture showed too low a stability.

In experiment 18 use was made of a catalyst mixture with an m>750 and a d <400 nm. The activity of this catalyst mixture was too low.

The only difference between the catalyst mixtures M, N and O is the weight ratio in which the $ZnO—Cr_2O_3$ composition and the silicate are present therein. The results of the experiments 13, 14 and 15 show that the activity of catalyst mixture N in which this ratio is 5, is higher than the activities of the catalyst mixtures M and O in which this ratio is 10 and 3.5, respectively.

## Claims

1. A process for the preparation of an aromatic hydrocarbon mixture, in which process an $H_2/CO$ mixture is contacted with a mixture of two catalysts of which one (catalyst X) has the capability of catalysing the conversion of an $H_2/CO$ mixture into acyclic oxygen-containing hydrocarbons and the other (catalyst Y) is a crystalline aluminium silicate which, after one hour's calcining in air at 500°C, has the following properties:

(a) thermally stable up to a temperature of at least 600°C,
(b) an X-ray powder diffraction pattern showing the reflections given in Table A.

TABLE A

| d(Å) | relative intensity | d(Å) | relative intensity |
|---|---|---|---|
| 11.1 | 100 | 3.84 (D) | 57 |
| 10.0 (D) | 70 | 3.70 (D) | 31 |
| 8.93 | 1 | 3.63 | 16 |
| 7.99 | 1 | 3.47 | 1 |
| 7.42 | 2 | 3.43 | 5 |
| 6.68 | 7 | 3.34 | 2 |
| 6.35 | 11 | 3.30 | 5 |
| 5.97 | 17 | 3.25 | 1 |
| 5.70 | 7 | 3.05 | 8 |
| 5.56 | 10 | 2.98 | 11 |
| 5.35 | 2 | 2.96 | 3 |
| 4.98 (D) | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |
| 4.00 | 4 | | |

(D)=doublet
$1 Å=10^{-10}$ m

(c) in the formula which represents the composition of the silicate, expressed in moles of the oxides, the $SiO_2/Al_2O_3$ molar ratio is above 200,
(d) an average crystallite size of less than 3000 nm, which crystalline aluminium silicate has been prepared by maintaining an aqueous mixture containing the following compounds: one or more compounds of an alkali metal (M), a tetrapropylammonium compound, one or more silicon

compounds and one or more aluminium compounds, at elevated temperature until the crystalline silicate has formed and subsequently separating this from the mother liquor and calcining it, characterized in that the crystalline aluminium silicate has been prepared from an aqueous mixture in which mixture the compounds are present in lhe following ratios, expressed in moles of the oxides:

$M_2O:SiO_2$=0.01—0.35,
$[(C_3H_7)_4N]_2O:SiO_2$=0.01—0.4,
$SiO_2:Al_2O_3$=150—1500, and
$H_2O:SiO_2$=5—65,

in that in the formula representing the composition of the silicate, the $SiO_2/Al_2O_3$ molar ratio is 200—750, and in that the silicate has an average crystallite size of 400—1500 nm.

2. A process according to claim 1, characterized in that the catalyst mixture contains per part by weight of catalyst Y, 2.5—12.5 parts by weight of catalyst X.

**Patentansprüche**

1. Ein Verfahren zur Herstellung einer Mischung aromatischer Kohlenwasserstoffe, in welchem Verfahren eine $H_2$/CO-Mischung mit einer Mischung aus zwei Katalysatoren kontaktiert wird, von welchen der eine (Katalysator X) die Eigenschaft hat, die Umwandlung einer $H_2$/CO-Mischung in acyclische sauerstoffhaltige Kohlenwasserstoff zu katalysieren, und der andere (Katalysator Y) ein kristillines Aluminiumsilikat ist, welches nach einstündigem Calcinieren an Luft bei 500°C die folgenden Eigenschaften aufweist:

(a) es ist bis zu einer Temperatur von mindestens 600°C stabil,
(b) es zeigt ein Röntgenpulverdiagramm mit den in Tabelle A angegebenen Beugungslinien

TABELLE A

| d(Å) | relative intensität | d(Å) | relative intensität |
|---|---|---|---|
| 11.1 | 100 | 3.84 (D) | 57 |
| 10.0 (D) | 70 | 3.70 (D) | 31 |
| 8.93 | 1 | 3.63 | 16 |
| 7.99 | 1 | 3.47 | 1 |
| 7.42 | 2 | 3.43 | 5 |
| 6.68 | 7 | 3.34 | 2 |
| 6.35 | 11 | 3.30 | 5 |
| 5.97 | 17 | 3.25 | 1 |
| 5.70 | 7 | 3.05 | 8 |
| 5.56 | 10 | 2.98 | 11 |
| 5.35 | 2 | 2.96 | 3 |
| 4.98 (D) | 6 | 2.86 | 2 |
| 4.60 | 4 | 2.73 | 2 |
| 4.35 | 5 | 2.60 | 2 |
| 4.25 | 7 | 2.48 | 3 |
| 4.07 | 2 | 2.40 | 2 |
| 4.00 | 4 | | |

(D)=Doublette
1Å=$10^{-10}$ m

(c) in der Formel, welche die Zusammensetzung des Silikats wiedergibt, ausgedrückt in Molen der Oxide, leigt das Molverhältnis von $SiO_2/Al_2O_3$ oberhalb 200,
(d) es hat eine Durchschnittskristallitgröße von weniger als 3000 nm, wobei das kristalline Aluminiumsilikat hergestellt worden ist, indem man eine wässrige Mischung, welche die folgenden Verbindungen enthält: eine oder mehrere Verbindungen eines Alkalimetalls (M), eine Tetrapropylammoniumverbindung, eine oder mehrere Siliciumverbindungen und eine oder mehrere Aluminiumverbindungen, auf erhöhter Temperatur hält, bis sich das kristalline Silikat gebildet hat, und trennt dieses anschließend von der Mutterlauge ab und calciniert es, dadurch gekennzeichnet, daß das kristalline Aluminiumsilikat aus einer wässrigen Mischung erhalten worden ist, in welcher Mischung die Verbindungen in den folgenden Verhältnissen, ausgedrückt als Mole der Oxide, vorliegen:

$M_2O:SiO_2=0,01—0,35$
$[(C_3H_7)_4N]_2O:SiO_2=0,01—0,4$
$SiO_2:Al_2O_3=150—1500$, und
$H_2O:SiO_2=5—65$

dab in der die Zusammensetzung des Silikats wiedergebenden Formel das Molverhältnis $SiO_2/Al_2O_3$ im Bereich von 200 bis 750 liegt und daß das Silikat eine Durchschnittskristalligröße von 400 bis 1500 nm aufweist.

2. Ein Verfahren gemäß Anspruch 1, dadurch gekennzeichnet, daß die Katalysatormischung je Gewichtsteil Katalysator Y 2,5 bis 12,5 Gewichtsteile Katalysator X enthält.

**Revendications**

1. Procédé de préparation d'un mélange hydrocarboné aromatique, dans lequel procédé on met en contact un mélange $H_2/CO$ avec un mélange de deux catalyseurs dont l'un (catalyseur X) a l'aptitude à catalyser la transformation d'un mélange $H_2/CO$ en hydrocarbures acycliques contenant de l'oxygène et l'autre (catalyseur Y) est un silicate d'aluminium cristallin qui, après une heure de calcination à l'air à 500°C, a les propriétés suivantes:

(a) stabilité thermique jusqu'à une température d'au moins 600°C,
(b) schéma de diffraction de poudre aux rayons X présentant les réflexions données au Tableau A.

TABLEAU A

| d(Å) | Intensité relative | d(Å) | Intensité relative |
|---|---|---|---|
| 11,1 | 100 | 3,84 (D) | 57 |
| 10,0 (D) | 70 | 3,70 (D) | 31 |
| 8,93 | 1 | 3,63 | 16 |
| 7,99 | 1 | 3,47 | 1 |
| 7,42 | 2 | 3,43 | 5 |
| 6,68 | 7 | 3,34 | 2 |
| 6,35 | 11 | 3,30 | 5 |
| 5,97 | 17 | 3,25 | 1 |
| 5,70 | 7 | 3,05 | 8 |
| 5,56 | 10 | 2,98 | 11 |
| 5,35 | 2 | 2,96 | 3 |
| 4,98 (D) | 6 | 2,86 | 2 |
| 4,60 | 4 | 2,73 | 2 |
| 4,35 | 5 | 2,60 | 2 |
| 4,25 | 7 | 2,48 | 3 |
| 4,07 | 2 | 2,40 | 2 |
| 4,00 | 4 | | |

(D)=doublet
$1Å=10^{-10}$ m

(c) dans la formule qui représente la composition du silicate, exprimée en moles des oxydes, le rapport molaire $SiO_2/Al_2O_3$ est supérieur à 200,
(d) une taille de cristallite moyenne inférieure à 3000 nm, lequel silicate d'aluminium cristallin a été préparé en maintenant un mélange aqueux contenant les composés suivants: un ou plusieurs composés d'un métal alcalin (M), un composé de tétrapropylammonium, un ou plusieurs composés de silicium et un ou plusieurs composés d'aluminium, a une temperature élevée jusqu'à formation du silicate cristallin, puis en séparant ceci de la liqueur-mère et en le calcinant, caractérisé en ce que le silicate d'aluminium cristallin a été préparé à partir d'un mélange aqueux, dans lequel mélange les composés sont présents dans les rapports suivants, exprimés en moles des oxydes:

$M_2O:SiO_2=0,01—0,35$
$[(C_3H_7)_4N]_2O:SiO_2=0,01—0,4$
$SiO_2:Al_2O_3=150—1500$, et
$H_2O:SiO_2=5—65$,

en ce que dans la formule représentant la composition du silicate le rapport molaire $SiO_2/Al_2O_3$ est de 200—750, et en ce que le silicate a une taille de cristallite moyenne de 400—1500 nm.

2. Procédé selon la revendication 1, caractérisé en ce que le mélange de catalyseur contient par partie pondérale de catalyseur Y de 2,5 à 12,5 parties en poids de catalyseur X.